# EUROPEAN PATENT APPLICATION

(11) **EP 4 173 591 A1**
(43) Date of publication of application: **03.05.2023**
(21) Application number: 22204601.3
(22) Date of filing: 30.10.2022
(51) Int. Cl.: A61B 90/00, A61C 1/16

(54) **PROTECTIVE SHIELD FOR A DENTAL INSTRUMENT**

(30) Priority: 01.11.2021 GR 20210100756
(71) Applicant: National Kapodistrian University of Athens, 10561 Athens (GR)
(72) Inventor: Kontakiotis, Evangelos, Athens (GR); Agrafioti, Anastasia, Anastasia (GR)
(74) Representative: Kouzelis, Dimitrios

(57) **Abstract**

Protective shield (1) for a dental instrument (10) during a dental procedure comprising a shield body (5) having a transparent main body region(2)and a secondary body region(3). The main body region (2) comprises an opening for receiving a dental instrument(10), and the secondary body region(3)extends at an angle from the main body region(2). Further, the present invention refers to a dental instrument arrangement (100) comprising such a protective shield (1) and a dental instrument (10).

## Description

The present invention refers to a protective shield for a dental instrument during a dental procedure.

During dental procedures, a large amount of airborne material may be produced. This aerosol material is often visible even to the naked eye, especially when a dental procedure being performed on a patient is observed from a close distance.

A so-called aerosol cloud is mostly produced during tooth preparation with a rotary instrument or air abrasion, the use of an air-water syringe, the use of an ultrasonic scaler and air polishing. The particles that form the aerosol cloud originate from the treatment site and the dental unit waterlines. This means in particular that the aerosol cloud contains particles from the patient's oral environment, from which bacteria and/or viruses can be spread in the environment in which the dental procedure takes place.

It should also not be overlooked that the oral cavity is part of the oronasal pharynx. So, bacteria and/or viruses originating from the nose, throat and respiratory tract are also harboured in the oral cavity of the patient. Among these, various pathogenic viruses and/or bacteria such as common cold and influenza viruses, herpes viruses, pathogenic streptococci and staphylococci, and the SARS virus may be present.

Thus, the dental procedures that have the potential to aerosolize saliva may cause airborne contamination of the environment of a dental clinic, so that several measures regarding the protection of the personnel's health as well as that of the patients should be taken. In particular, the aerosols created by the interaction of coolant water and ultrasonic vibrations or by compressed air and a rotary should be controlled to the greatest extent possible.

The use of personal barrier protection such as masks, gloves and eye protection eliminate much of the danger inherent in splatter droplets arising from the operative site. Nevertheless, there is room of improvement regarding the protection of the individuals during a dental procedure.

Towards this end, a protective shield for a dental instrument during a dental procedure is proposed by the present invention. The protective shield serves as a barrier for aerosol material produced during a dental procedure.

The protective shield for a dental instrument during a dental procedure comprises a shield body having a transparent main body region and a secondary body region. The main body region comprises an opening for receiving a dental instrument.

Advantageously, the secondary body region extends at an angle from the main body region. In other words, the secondary body region is inclined at an angle with respect to the main body region. It is noted that the formulation "an angle" may comprise one single angle or two or more different angles.

The protective shield according to the present invention can be applied to the everyday clinical practice of dentists and thus minimize the aerosols that are diffused in the dental clinic, providing effective protection. In particular, the present invention can be an effective adjunct for the protection of a dentist during every day clinical practice as well as the suppression of a virus transmission in the dental office. The protective shield according to the present invention can be easily manufactured and is a low-cost product, so that it can become part of the personal protective equipment used in everyday dental clinical practice.

The advantages of the present invention are particularly important in case of a pandemic, as it is evident that dental procedures and especially those that generate aerosols have to be provided with extreme caution in order to protect both the dental personnel as well as all the patients that visit a dental office each day. Especially, the provision of the secondary body region is advantageous, as it defines a space, which helps trap aerosol material produced during a dental procedure or reduce their speed, compared to the case where no secondary body region is provided. Further, the transparent design of the main body region allows the dentist to see the oral cavity of the patient when performing a dental procedure on the patient.

Within the context of the present invention, the main body region can be characterized as a first body region and the secondary body region as a second body region.

Preferably, when both the main body region and the secondary body region are projected on the same plane, the main body region has a larger surface area than the secondary body region. However, it is also possible that the main body region has a smaller surface area than the secondary body region, when both the main body region and the secondary body region are projected on the same plane.

Preferably, the main body region is an inner body region and the secondary body region an outer body region.

The protective shield is preferably flexible. Thus, the protective shield will not bother or hurt the patient if it is pressed against the patient's mouth during the dental procedure.

Preferably, the secondary body region is transparent. Thus, the dentist is able to see the oral cavity of the patient also through the secondary body region when performing a dental procedure on the patient.

Preferably, the secondary body region extends outwards from the main body region. The formulation that the secondary body region extends outwards from the main body region means in particular that the secondary body region extends away from the main body region.

Preferably, the main body region has anti-reflection properties. It is further preferred that also the secondary body region has anti-reflection properties. Thus, the dentist may have a clear view of the oral cavity during the dental procedure, even under the strong light of a dental unit used during the dental procedure.

According to an advantageous embodiment of the present invention, the main body region has a circular shape, preferably with a diameter equal to or larger than 40 mm, more preferably equal to or larger than 50 mm, more preferably equal to or larger than 60 mm, more preferably equal to or larger than 70 mm.

According to an alternative advantageous embodiment of the present invention, the main body region has a rectangular shape, preferably with a side length equal to or larger than 40 mm, more preferably equal to or larger than 50 mm, more preferably equal to or larger than 60 mm, more preferably equal to or larger than 70 mm. The term "side length" refers to the length of any of the sides of the rectangular shape. In particular, the main body region may have the shape of a square. Within the context of the present invention, the term "rectangular shape" may preferably also comprise a rectangular shape with at least one cut edge.

Due to the described dimensioning of the main body region, it can be made sure that the majority of the aerosol material produced during a dental procedure will be blocked by the protective shield. In addition, the main body region is large enough to cover at least a part of the mouth opening of a patient during a dental procedure.

Preferably, the main body region is formed as a sheet or plate. The terms "sheet" or "plate" means in particular that a thickness of the main body region is much smaller than the other dimension(s) of the main body region. For example, if the main body region has a circular shape, the aforementioned terms mean that the thickness of the main body region is much smaller than its diameter. Accordingly, if the main body region has a rectangular shape, the aforementioned terms mean that the thickness of the main body region is much smaller than a length of its sides.

Preferably, the main body region has a main surface with a surface area of at least 1100 mm2. Thus, the main body region is large enough to cover at least a part of the mouth opening of a patient during a dental procedure.

Preferably, the secondary body region is formed such that it defines/encloses a space with a maximum cross-sectional area larger than a surface area of a main surface of the main body region. This means in particular that the protective shield is wider in the area of the secondary body region than in the area of the main body region.

According to an advantageous embodiment, the secondary body region extends at a single angle from the main body region. In this case, the secondary body region may be continuously formed or comprise sub regions. Here, preferably, the main body region is flat and the angle, at which the secondary body region extends from the main body region, may be 90 degrees. In other words, the secondary body region may extend vertically from the main body region. Alternatively, the main body region is flat and the angle, at which the secondary body region extends from the main body region, may advantageously be larger than 90 degrees and lower than 180 degrees, wherein the secondary body region extends outwards from the main body region. In other words, the angle at which the secondary body region extends from the main body region is at least 90 degrees and less than 180 degrees. The angle is measured from the plane of the main body region.

According to an alternative advantageous embodiment of the present invention, the secondary body region may comprise at least two sub regions, in particular a plurality of sub regions, that extend at different angles from the main body region and/or at least two sub regions, in particular a plurality of sub regions, from which a first sub region extends from the main body region at the angle, which is a first angle, and a second sub region extends from the first sub region at a second angle. It is understood that the second angle is different from the first angle. Preferably, the main body region is flat and the first angle, at which the first sub region extends from the main body region is 90 degrees or larger than 90 degrees and smaller than 180 degrees. The second angle is preferably 90 degrees or larger than 90 degrees and smaller than 180 degrees, wherein the second sub region extends outwards, in other words away, from the first sub region. Both the first angle and the second angle are measured from the plane of the main body region.

Preferably, the secondary body region extends over a whole circumference of the main body region. In case of a second sub region extending at an angle from a first sub region, the first sub region and/or the second sub region may extend over the whole circumference of the main body region.

The main body region and/or the secondary body region is/are preferably made of plastic, in particular PET.

The main body region and/or the second body region preferably has/have a thickness of 0.01 mm to 0.03 mm, preferably 0.02 mm.

The opening preferably has a dimension between 10 mm and 12 mm, in particular 11 mm.

Preferably, the opening has a cross-sectional area between 75 mm2 and 115 mm2.

Preferably, the opening is circular. In this case, the aforementioned dimension of the opening corresponds to a diameter of the opening.

Due to the aforementioned dimensioning of the opening, the protective shield can be mounted to the dental instrument, in particular to a shaft thereof, at a position, at which, an effective blocking of the aerosol material produced during the aforementioned dental procedures can be achieved.

The protective shield preferably comprises a mounting means, by which the protective shield is mountable to the dental instrument. The mounting means preferably comprises at least one flexible, in particular silicone, tube. More preferably, the mounting means comprises two flexible- in particular silicone, tubes. The tube(s) is/are suitable for medical/pharmaceutical applications. The design of the mounting means as at least one flexible tube has the advantage of an easy and stable fixation of the protective shield to the dental instrument. In addition, the flexible tube(s) can serve as a sealing element sealing against the shaft of the dental instrument. Thus, droplets produced during the dental procedure cannot escape or are prevented from escaping through the mounting site between the protective shield and the dental instrument.

The tube(s) may preferably have an inner diameter of 5 mm to 7 mm and/or an outer diameter of 7 mm to 9 mm. Here, a material thickness of the tube(s) is preferably at least 1 mm, preferably at least 2 mm.

More preferably, the tube(s) may have an inner diameter of 6 mm and an outer diameter of 8 mm. Here, the material thickness is 2 mm.

Additionally, the protective shield may optionally receive an adhesive strip or a tablet of antibacterial or disinfectant, aiming at the suppression or the minimizing of the bacterial activity of the produced aerosol. In order to receive the antibacterial tablet, the shield may comprise a small recess of a size able to receive a pill or tablet. The recess is open on the side of the shield which faces the dentist, in order to receive the tablet and has a plurality of holes or openings on the side of the shield which faces the patient, in order to facilitate the diffusion of the disinfectant in the area of the mouth. The shield itself provides a quantitative protection against the diffusion of the bacterial activity, whilst the addition of the disinfectant contributes to the qualitative suppression of the produced aerosol.

The present invention further refers to a dental instrument arrangement that comprises a dental instrument and a previously described protective shield.

The dental instrument arrangement may be in the form of a kit comprising the dental instrument and the protective shield.

Preferably, the protective shield is arranged on the dental instrument, in particular on a shaft of the dental instrument.

The dental instrument may preferably be a rotary instrument, a dental air polisher, a dental handpiece, an air-water syringe, a scaler, in particular an ultrasonic scaler, a dental burnisher or a dental excavator.

The protective shield of the present invention can be applied to the everyday clinical practice of dentists and thus minimize the aerosols that are diffused in the dental clinic, providing effective protection to the dentist, assistants, and patients. In particular, the present invention can be an effective adjunct for the protection of a dentist during every day clinical practice as well as the suppression of a virus transmission in the dental office.

These and further details, advantages and features of the present invention will be described based on embodiments of the invention and by taking reference to the accompanying figures. Identical or equivalent elements and elements which act identically or equivalently are denoted with the same reference signs. Not in each case of their occurrence a detailed description of the elements and components is repeated.
Figure 1 shows a simplified schematic perspective view of a dental arrangement comprising a protective shield according to a first embodiment of the present invention and a dental instrument, on which the protective shield is arranged.
Figure 2 shows a simplified schematic perspective view of the dental arrangement of figure 1 during a dental procedure.
Figure 3 shows a simplified schematic front view of a protective shield according to a second embodiment of the present invention.
Figure 4 shows a simplified schematic front view of a protective shield according to a third embodiment of the present invention.
Figure 5 shows an additional feature which can be adapted to any form of the protective shield of the present invention.

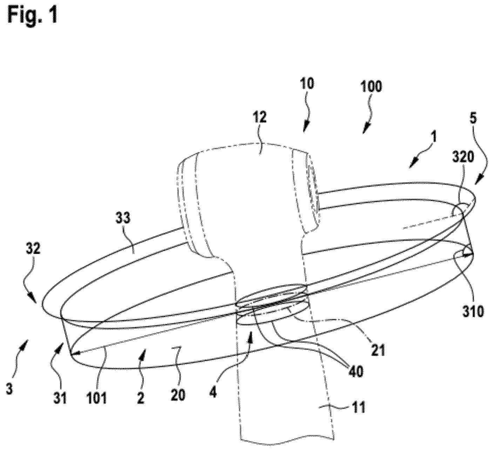

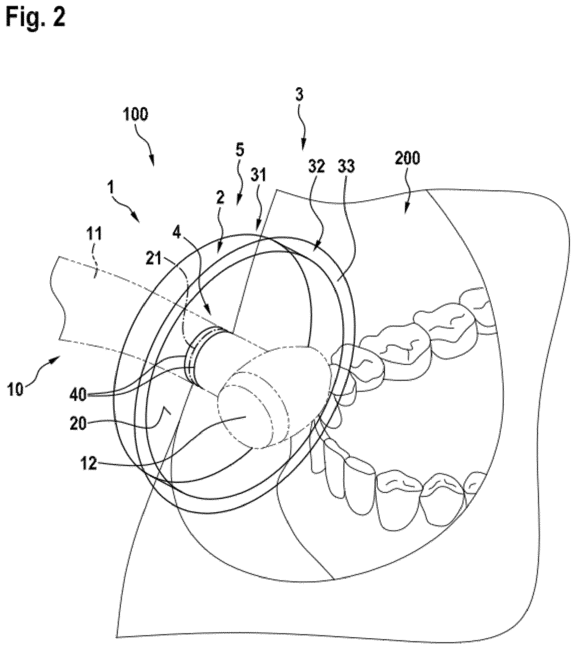

In the following, a dental instrument arrangement 100 having a dental instrument 10 and a protective shield 1 according to a first embodiment of the present invention is described in detail with reference to figures 1 and 2.

In the present embodiment, the dental instrument 10 is a dental handpiece having a shaft 11 and a head 12. However, any dental instrument, in particular those which produce aerosol material during a dental procedure, can be used together with the protective shield 1. In figure 2, the dental instrument arrangement 100 is shown during a dental procedure performed in the mouth cavity 200 of a dental phantom or dental training manikin.

The protective shield 1 comprises a shield body 5, which is formed as one piece and has a transparent main body region 2 and a transparent secondary body region 3. The main body region 2 comprises an opening 21 for receiving the dental instrument 10 and is flat (flat-shaped).

Both the main body region 2 and the secondary body region 3 are made of plastic, in particular PET, and each have a thickness of 0.01 mm to 0.03 mm, preferably 0.02 mm. The protective shield 1 is flexible, so that it will not bother the patient if it is pressed against the patient's mouth during the dental procedure.

Further, both the main body region 2 and the secondary body region 3 have anti-reflection properties. Thus, a clear view of the mouth cavity of the patient through the main body region 2 and the secondary body region 3 can be achieved, even under the strong light of a dental unit used during the dental procedure.

As can be seen from the figures, the main body region 2 is an inner body region and the secondary body region 3 an outer body region of the shield body 5. Further, the secondary body region 3 extends at an angle outward from the main body region 2. In particular, the secondary body region 3 comprises a first sub region 31 and a second sub region 32. The first sub region 31 extends at a first angle 310 from the main body region 3, wherein the second sub region 32 extends at a second angle 320 from the first sub region 31. In this embodiment, the first angle 310 is 90 degrees, when measured from the plane of the main body region 2. However, the first angle 310 may also be larger than 90 degrees and smaller than 180 degrees, when measured from the plane of the main body region 2. The second angle 320 is larger than 90 degrees and smaller than 180 degrees, when measured from the plane of the main body region 2 (or a plane parallel to the plane of the main body region 2). It is noted that it is also possible that the secondary body region 3 comprises only the first sub region 31.

Advantageously, the secondary body region 3 extends, more specifically the first sub region 31 and the second sub region 32 extend, over a whole circumference of the main body region 2.

It can further be seen from the figures that the secondary body region 3 is formed such that it defines/encloses a space 33 with a maximum cross-sectional area larger than a surface area of a main surface 20 of the main body region 2.

The main body region 2 has in this embodiment a circular shape with a diameter 101 equal to or larger than 40 mm, more preferably equal to or larger than 50 mm, more preferably equal to or larger than 60 mm, more preferably equal to or larger than 70 mm.

The opening 21 provided in the main body region 2 is of circular shape and has a diameter between 10 mm and 12 mm, in particular 11 mm. Due to this size of the opening 21, the protective shield 1 can be mounted to the dental instrument 10 at a position close to the head 12 (distal end) of the dental instrument 10 and thus also close to the mouth cavity 200. Thus, aerosol material produced during a dental procedure can be prevented from exiting the mouth cavity 200.

For mounting the protective shield 1 to the shaft 11 of the dental instrument 10, the protective shield 1 comprises a mounting means 4. The mounting means 4 comprises two silicone tubes 40 that are suitable for medical/pharmaceutical applications and have an inner diameter of 5 mm to 7 mm and an outer diameter of 7 mm to 9 mm.

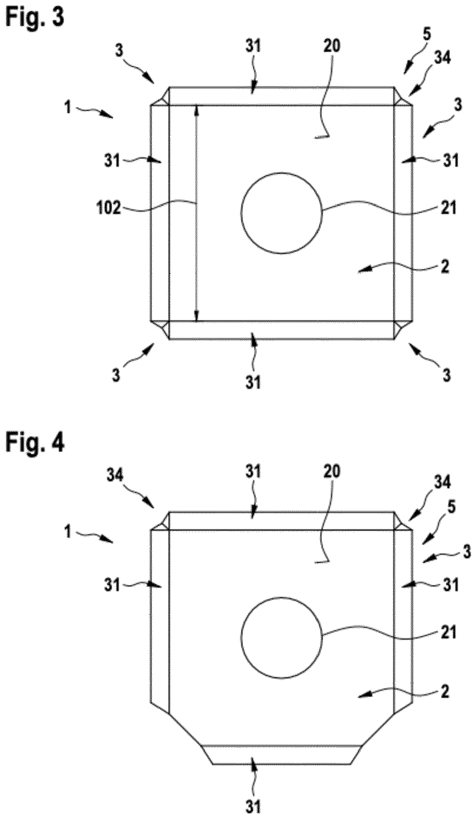

Figure 3 refers to a protective shield 1 according to a second embodiment of the present invention, which can be used together with the dental instrument 10 shown in figures 1 and 2.

The difference between the protective shield 1 according to the second embodiment and that according to the first embodiment lies in the design of the main body region 2 and of the secondary body region 3.

Here, as can be seen from figure 3, the main body region 2 has the shape of a square with a side length 102 of at least 40 mm, more preferably at least 50 mm, more preferably at least 60 mm, more preferably at least 70 mm.

Furthermore, the secondary body region 3 of the protective shield 1 according to the second embodiment comprises a plurality of sub regions 31 that each extends from the main body region 2 at an angle larger than 90 degrees and smaller than 180 degrees, when measured from the plane of the main body region 2. In the present embodiment, all sub regions 31 extend at the same angle. It is also possible that the sub regions 31 extend from the main body region 2 at different angles from each other, each angle being at least 90 degrees and less than 180 degrees.

Each sub region 31 is connected to its neighbouring sub regions 31 by a corresponding connection region 34. However, it is also possible that a sub region 31 is separately formed from its/their neighbouring sub regions 31. In this case, no connection regions 34 are provided for the two neighbouring sub regions 31 that are not connected to each other. For the sake of comparison, it is noted that each of the sub region 31 and sub region 32 of the secondary body region 3 in the protective shield 1 of the first embodiment is formed as a single region.

The shield body 5 can be characterized in the present embodiment as having bent edges, wherein the bent edges correspond to the secondary body region 3, in particular the sub regions 31.

Figure 4 refers to a protective shield 1 according to a third embodiment of the present invention, which, similar to the protective shield 1 according to the second embodiment, can also be used together with the dental instrument 10 shown in figures 1 and 2.

The protective shield 1 according to the third embodiment differs from that according to the second embodiment in that not each of the sub regions 31 is connected to all of its neighbouring sub regions 31. For example, the upper sub region 31 is connecting to both neighbouring sub regions 31 by corresponding connection regions 32 and the left and right sub regions 31 are each connected only to the upper sub region 31. The lower sub region 31 is not connected to any of its neighbouring sub regions 31 (left and right).

Here, the main body region 2 is also formed in the shape of a rectangular with two cut edges. This offers better or larger volume of flow of the airborne material than the protective shield 1 of the embodiment of figure 3.

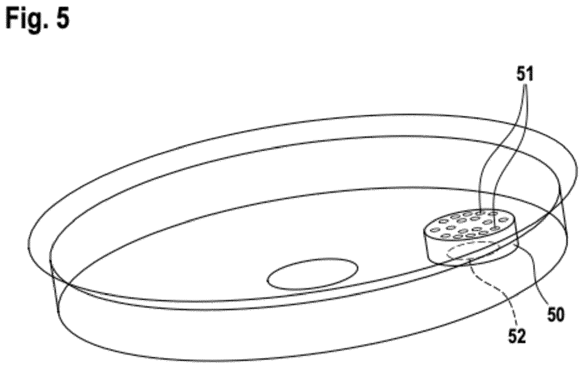

Figure 5 shows an additional and optional feature which can be adapted to any form of the protective shield of the present invention, consisting of a recess 50. The recess 50 is able to receive an antibacterial tablet (not shown in the figure), which is inserted from a hole 52 on the side of the shield facing the dentist. On the opposite to this side, which is the side facing the patient, the recess has a plurality of holes 51 aiming at facilitating the diffusion of the antibacterial material to the operating area.

Alternatively, one or more antibacterial strips or tablets can be self-adhered on the side of the shield facing the patient and provide an additional qualitative suppression of the bacterial activity of the produced aerosol.

The antibacterial strips or the disinfectant tablets, can be of a known composition comprising for example, %w/w: Sodium Benzoate ^{~} 5.23; Menthol - 0.14; Thymol ^{~} 0.13 or any other composition known in the art.

In addition to the foregoing description of the present invention, for an additional disclosure explicit reference is taken to graphic representation of figures 1 to 5.

## Claims

1. A protective shield (1) for a dental instrument(10) during a dental procedure, comprising:
- a shield body (5) having a transparent main body region(2)and a secondary
- body region(3),
- wherein:
- the main body region (2) comprises an opening(21)for receivinga dental instrument(10), and
- the secondary body region (3) extends at an angle(310, 320)from the main body region(2).

2. The protective shield(1)according to claim 1, wherein:
- the secondary body region (3) is transparent,and/or
- wherein the main body region (2) and in particular also the secondary body region (3) has/have anti-reflection properties, and/or
- the secondary body region (3) extends outwards from the main body region(2).

3. The protective shield (1)according to claim1 or 2, wherein the secondary body region (3) extends at a single angle (310) from the main body region (2), preferably wherein the main body region (2) is flat and the angle (310) at which the secondary body region (3) extends from the main body region (2)is 90 degreesor larger than 90 degreesand lower than 180 degrees, wherein the secondary body region (3) extends outwards from the main body region (2).

4. The protective shield (1)according to any of thepreceding claims, wherein the main body region(2)has a circular shape, preferably with adiameter equal to or largerthan 40 mm, or wherein the main body region (2) has a rectangular shape, preferably with a side length equal to orlarger than 40 mm.

5. The protective shield (1) according to any of the preceding claims, wherein:
- the main body region (2) is formed as a sheet, and/or
- the main body region (2) has a main surface (20) with a surface area of at least 1100 mm2, and/or
- wherein the secondary body region (3) is formed such that it defines a space (33) with a maximum cross-sectional area larger than a surface area of a main surface (21) of the main body region (2).

6. The protective shield (1) according to any of claims 1, 2 and 4 to 6, wherein the secondary body region (3) comprises at least two sub regions (31) that extend at different angles from the main body region (2) and/or at least two sub regions (31), from which a first sub region (31) extends from the main body region (2) at a first angle (310) and a second sub region (32) extends from the first sub region (31) at a second angle (320).

7. The protective shield (1) according to any of the preceding claims, wherein the secondary body region (3) extends over a whole circumference of the main body region (2).

8. The protective shield (1) according to any of the preceding claims, wherein:
- the main body region (2) and/or the secondary body region (3) is/are made of plastic, in particular PET, and/or
- the main body region (2) and/or the second body region (3) has/have a thickness of 0,01 mm to 0,03 mm, preferably 0,02 mm.

9. The protective shield (1) according to any of the preceding claims,
- wherein the opening (21) has a dimension between 10 mm and 12 mm
- and/or a cross-sectional area between 75 mm² and 115 mm²,
- and/or comprising a mounting means (4), by which the protective shield (1) is mountable to the dental instrument (10), wherein the mounting means (4) preferably comprises at least one flexible, in particular silicone, tube (40), more preferably two such tubes (40).

10. The protective shield (1) according to any of the preceding claims, comprising at least one antibacterial strip or tablet, which is adhered on the side of the shield (1) facing the patient.

11. The protective shield (1) according to any of the preceding claims, comprising a recess (50) for receiving an antibacterial tablet, said recess (50) comprising holes (51) for facilitating the diffusion of the antibacterial material to the operating area.

12. The protective shield (1) according to any of the preceding claims, comprising a hole (52) for receiving an antibacterial tablet recess (50) in the recess.

13. A dental instrument arrangement (100), comprising:
- a dental instrument (10), and
- a protective shield (1) according to any of the preceding claims,
preferably, wherein the protective shield (1) is arranged on the dental instrument (10), in particular on a shaft (11) of the dental instrument (10), and/or
wherein the dental instrument (10) is a rotary instrument, a dental air polisher, a dental handpiece, an air-water syringe, a scaler, in particular an ultrasonic scaler, a dental burnisher or a dental excavator.
